# EUROPEAN PATENT APPLICATION

(11) **EP 4 710 916 A1**
(43) Date of publication of application: **18.03.2026**
(21) Application number: 24382988.4
(22) Date of filing: 16.09.2024
(51) Int. Cl.: A61K 8/44, A61K 31/198, A61Q 11/00

(54) **COMPOSITION COMPRISING GLUTAMATE FOR CARIES PREVENTION**

(71) Applicant: Fundación para el Fomento de la Investigación Sanitaria y Biomédica de la Comunitat Valenciana (FISABIO), 46020 Valencia (ES)
(72) Inventor: MIRA OBRADOR, Alejandro, 46020 Valencia (ES); ROSIER, Bob Thadeus, 46020 Valencia (ES); NADAL RUIZ, María, 46020 Valencia (ES)
(74) Representative: Manuel Illescas y Asociados, S.L.U.

(57) **Abstract**

Oral composition comprising glutamate, or an amine, solvate or hydrate thereof, for use in the treatment and/or prevention of caries, wherein the composition is orally administered and increases the pH of the saliva, reducing the risk of caries development. Said oral composition increases the amount of health-associated bacteria and/or decreases the amount of disease-associated bacteria and is useful for the treatment and/or prevention of oral diseases, such as periodontal diseases, halitosis and diseases caused by *Candida.* Method to determine the response of a mammal to the treatment with glutamate, or with an amine, solvate or hydrate thereof, comprising determining in saliva the amount of health-associated or disease-associated bacteria or a dysbiosis index, and identifying the mammal as responsive or non-responsive to the treatment.

## Description

### TECHNICAL FIELD

The present invention relates to oral compositions for use in the treatment and prevention of oral diseases including caries, periodontal diseases, halitosis, and oral diseases caused by *Candida.* Particularly, the present invention relates to oral compositions comprising glutamate, or an amine, solvate or hydrate thereof.

### BACKGROUND ART

Dental caries is the most prevalent chronic disease of humanity, as well as the most common cause of tooth loss and pain in the oral cavity.

The oral cavity is a diverse and unique ecosystem in the human body with hundreds of bacterial species. Dental plaque is formed when oral microorganisms adhere to the tooth surface and form biofilms. Frequent consumption of carbohydrates, especially sugars, can lead to a change in the composition of these biofilms, known as dysbiosis, by selectively increasing saccharolytic (carbohydrate consuming), acidogenic (i.e., acid producing) and acid tolerant species. These species can ferment carbohydrates into organic acids, such as lactic acid and, to a lesser extent, acetic acid and propionic acid. These organic acids can lower the pH below a critical value (around pH 5.5), leading to demineralisation of tooth enamel and thus to the development of dental cavities.

Prebiotics are compounds that are selectively utilised by host microorganisms conferring a health benefit. Prebiotics have been used to treat and prevent dysbiosis by promoting the growth, selective advantage and/or beneficial metabolism of health-associated oral bacteria. Anti-caries effects of prebiotics include the inhibition of cariogenic bacteria and pH buffering effects. An example of a prebiotic against caries is arginine, which is metabolized by oral bacteria leading to ammonia production in dental plaque, thus, increasing the pH.

Periodontal diseases include gingivitis, periodontitis and periimplantitis. Gingivitis (i.e., gum inflammation) is a common response to dental plaque accumulation that can increase the risk of periodontitis in susceptible individuals. Periodontitis is a chronic and destructive inflammation that leads to the formation of a periodontal pocket. Over time, periodontitis can lead to tooth loss and increases the risk of systemic complications, such as cardiovascular diseases, pregnancy complications or diabetes, among others. In periodontitis, the levels of anaerobic, inflammation-tolerant and proteolytic bacteria increase and contribute to the destructive inflammatory response. On a microbial level, prebiotics that can limit periodontitis inhibit periodontitis-associated species and/or stimulate the growth of periodontal health-associated species. Periimplantitis is another periodontal disease, which is similar to periodontitis, but affects implants instead of natural teeth.

Periodontitis is associated with halitosis and different bacterial species are associated with both conditions. Halitosis mostly results from dysbiotic changes of the tongue microbiota, which result in an increase of volatile sulphur compound (VSC) production. Additionally, periodontal pockets of periodontitis patients can contribute to VSC production and halitosis. Anti-halitosis prebiotics inhibit VSC production or decrease the levels of halitosis-associated bacterial species.

There is a need to provide oral compositions for the treatment and prevention of caries and to provide prebiotics for the treatment and prevention of biofilm-mediated diseases including periodontal diseases, halitosis, and oral diseases caused by *Candida.*

### SUMMARY OF INVENTION

As used herein, the term "glutamate" refers to the anionic form of glutamic acid and of any salt, amine, solvate or hydrate thereof. For the purpose of the present description, the term "glutamate" includes glutamic acid.

As used herein, the term "prebiotic" refers to a compound that is selectively utilised by host microorganisms conferring a health benefit.

The problem to be solved can be seen in the provision of an oral composition for the treatment and prevention of caries.

In addition, the problem to be solved is to provide a prebiotic for the treatment and prevention of an oral disease selected from periodontal diseases, halitosis, and oral diseases caused by *Candida.*

The present invention solves the technical problem by an oral composition comprising glutamate, or an amine, solvate or hydrate thereof, that increases the pH of the saliva of the mouth.

The present invention provides an oral composition comprising glutamate, for use in the treatment and/or prevention of caries in a mammal, wherein the composition is orally administered to the mammal and, unexpectedly, increases the pH of the saliva of the mouth of the mammal, thereby, reducing the risk of caries development in the mammal.

In embodiments of the composition for use of the invention, the mammal is a human.

In embodiments, the present invention refers to a method for the treatment and/or prevention of caries in a mammal, the method comprising orally administering an oral composition comprising glutamate, or an amine, solvate or hydrate thereof, to said mammal, wherein the composition surprisingly increases the pH of the saliva of the mouth of the mammal, thereby, reducing the risk of caries development in the mammal.

In embodiments, the present invention refers to the use of an oral composition comprising glutamate, or an amine, solvate or hydrate thereof, for the manufacture of an oral care product for the treatment and/or prevention of caries in a mammal, wherein the composition is orally administered to the mammal and, contrary to what might be expected, increases the pH of the saliva of the mouth of the mammal, thereby, reducing the risk of caries development in the mammal.

The present invention also provides an oral composition comprising glutamate, or an amine, solvate or hydrate thereof, for use in the treatment and/or prevention of an oral disease in a mammal, wherein the composition is orally administered to the mammal and the oral disease is selected from caries, periodontal disease, halitosis, and oral disease caused by *Candida.*

In embodiments, the present invention refers to a method for the treatment and/or prevention of an oral disease in a mammal, the method comprising orally administering an oral composition comprising glutamate, or an amine, solvate or hydrate thereof, to said mammal, wherein the oral disease is selected from caries, periodontal disease, halitosis, and oral disease caused by *Candida.*

In embodiments, the present invention refers to the use of an oral composition comprising glutamate, or an amine, solvate or hydrate thereof, for the manufacture of an oral care product for the treatment and/or prevention of an oral disease in a mammal, wherein the composition is orally administered to the mammal and the oral disease is selected from caries, periodontal disease, halitosis, and oral disease caused by *Candida.*

In preferred embodiments, periodontal disease is selected from the group consisting of gingivitis, periodontitis and periimplantitis.

In preferred embodiments, the mammal is a human.

In the present invention, it has been found that glutamate limits a pH drop when sugars are fermented by oral bacteria, thereby, reducing the risk of caries development. This effect of glutamate is produced by the consumption of protons during the decarboxylation of L-glutamate to the non-protein amino acid γ-aminobutyric acid (GABA) by the enzyme glutamate decarboxylase (GAD). The proton consumption in the reaction contributes to lower the acidification caused by caries-producing bacteria. In the present invention, it has been found that the glutamate decarboxylase (GAD) is present in oral bacteria. The decarboxylation of L-glutamate to GABA by the glutamate decarboxylase (GAD) is shown in **Scheme 1:**

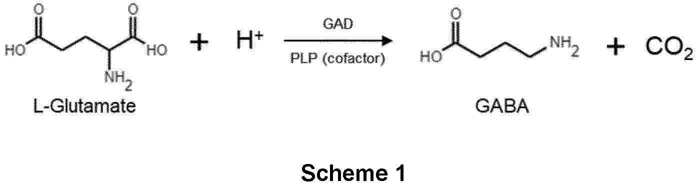

In **Scheme 1,** PLP refers to pyridoxal 5'-phospate.

The examples of the present application show that, surprisingly, monosodium glutamate acts as a prebiotic for the oral microbiota to prevent caries through pH buffering, an effect that would be also obtained by any other glutamate salt, by glutamic acid, or by any other form of glutamate (e.g., oligopeptides or peptides comprising glutamate). Unexpectedly, the examples of the present application show that glutamate increases the pH of the saliva in the mouth.

Alternatively, to any form of glutamate, glutamine (amine of glutamate) can be used as a source of glutamate. Specifically, bacteria convert glutamine into glutamate, releasing ammonia (as shown in **Scheme 2**), having an additional pH buffering effect on top of the pH buffering effects of glutamate.

Glutamine + H₂O → Glutamate + NH₃ **Scheme 2**

In embodiments of the composition for use of the invention, the glutamate is selected from the group consisting of monosodium glutamate, potassium glutamate, calcium glutamate, magnesium glutamate, and ammonium glutamate.

In embodiments of the composition for use of the invention, the amine is glutamine.

In embodiments of the composition for use of the invention, the concentration of glutamate, or of the amine, solvate or hydrate thereof, in the composition, is from 10 to 220 mmol/kg.

In embodiments of the composition for use of the invention, the concentration of glutamate, or of the amine, solvate or hydrate thereof, in the composition, is from 10 to 70 mmol/kg. Preferably, the concentration of glutamate, or of the amine, solvate or hydrate thereof, in the composition, is: from 30 to 190 mmol/kg, or from 50 to 170 mmol/kg, or from 70 to 150 mmol/kg, or from 50 to 130 mmol/kg, or from 70 to 110 mmol/kg, or from 80 to 100 mmol/kg.

In preferred embodiments of the composition for use of the invention, the glutamate is monosodium glutamate monohydrate and the concentration of monosodium glutamate monohydrate in the composition is from 2 to 36 g/kg. Preferably, monosodium glutamate monohydrate concentration is: from 6 to 32 g/kg, or from 10 to 28 g/kg, or from or from 14 to 24 g/kg, or from 18 to 20 g/kg.

In preferred embodiments of the composition for use of the invention, the dose of glutamate, or of the amine, solvate or hydrate thereof, is at least 5.3 µmol.

In preferred embodiment of the composition for use of the invention, the glutamate is monosodium glutamate monohydrate and the concentration of monosodium glutamate monohydrate in the composition is and the amount of monosodium glutamate monohydrate is at least 1 mg.

In embodiments of the composition for use of the invention, the composition increases the pH by consuming protons in decarboxylation reaction of glutamate to produce γ-aminobutyric acid catalysed by the enzyme glutamate decarboxylase.

The changes induced by glutamate in the microbiota composition, which are shown in the examples of the present application, are beneficial for oral health, including an increase in at least one health-associated bacterium selected from the group consisting of *Haemophilus, Veillonella* and *Actinomyces* (all commensal bacteria associated with periodontal health and/or the absence of halitosis), and/or decreasing at least one disease-associated bacterium selected from the group consisting of *Treponema, Catonella, Filifactor, Prevotella, Dialister, Alloprevotella, Megasphaera* and *Porphyromonas* (periodontal diseases/halitosis-associated bacteria); and/or at least one disease-associated bacterium selected from *Atopobium* and *Oribacterium* (caries-associated bacteria).

The changes induced by glutamate include increasing the amount of health-associated bacteria in oral biofilms of the mammal, and said health-associated bacteria may reduce the growth of *Candida.* Therefore, the oral composition of the invention is for use in the treatment and/or prevention of oral disease caused by *Candida.*

The changes induced by glutamate in the microbiota composition, which are shown in the examples of the present application, are beneficial for periodontal health and halitosis prevention, meaning that they are beneficial to prevent periodontal diseases and halitosis, by decreasing the amount of dysbiosis from a periodontitis perspective, which can be determined by calculating validated dysbiosis indexes, such as the Subgingival Microbial Dysbiosis Index, or determining the amount of at least one health-associated bacterium in oral biofilms of the mammal or of at least one disease-associated bacterium in oral biofilms of the mammal. The amount of said bacterium may be determined by qPCR, sequencing, cultivation or by any other method known in the art.

In the examples of the present application, the effect of glutamate was studied *in vitro* with samples of human oral microbiota, therefore, in conditions which take into account the activity of the complex and entire microbial community comprising hundreds of bacterial species. As far as known by the inventors, this is the first study of the effect of glutamate on the composition of the complex and entire microbial community of the human oral microbiota. Studying the effect of glutamate in complex and entire microbial communities is the only way to reliably and robustly assess how the glutamate affects bacteria of said communities. Studies with just one or a few bacteria do not provide reliable and robust insights about the effect of glutamate over the complex and entire microbial community of the human oral microbiota.

Therefore, in embodiments of the composition for use of the invention, the composition further increases the amount of health-associated bacteria in oral biofilms of the mammal and/or decreases the amount of disease-associated bacteria in oral biofilms of the mammal and the composition is useful for the treatment and/or prevention of an oral disease in the mammal, the oral disease being selected from periodontal disease and halitosis.

In embodiments of the composition for use of the invention, the health-associated bacterium is selected from the group consisting of *Haemophilus haemolyticus, Haemophilus pittmaniae, Haemophilus parainfluenzae, Haemophilus* spp., *Actinomyces* spp., *Veillonella rogosae, Veillonella dispar,* and *Veillonela* spp.; and/or disease-associated bacterium is selected from the group consisting of *Porphyromonas endodontalis, Porphyromonas* spp., *Treponema* spp., *Catonella morbi, Catonella* spp., *Filifactor alocis, Filifactor* spp*., Prevotella intermedia, Prevotella* spp., *Dialister invisus, Dialister* spp., *Absconditabacteriales* (SR1) spp., *Porphyromonas endodontalis, Porphyromonas* spp., *Alloprevotella tannerae, Alloprevotella* spp., *Megasphaera micronuciformis, Megasphaera* spp., *Atopobium parvulum, Atopobium* spp., *Oribacterium parvum,* and *Oribacterium spp.*

In embodiments of the composition for use of the invention, the health-associated bacterium is selected from the group consisting of *Haemophilus haemolyticus, Haemophilus pittmaniae, Haemophilus parainfluenzae, Haemophilus* spp., *Actinomyces* spp., *Veillonella rogosae, Veillonella dispar,* and *Veillonela* spp ; and/or the disease-associated bacterium is selected from the group consisting of *Treponema* spp., *Catonella* spp., *Filifactor* spp., *Prevotella* spp, *Dialister* spp*., Alloprevotella* spp., *Megasphaera* spp. *Porphyromonas* spp. (periodontal diseases/halitosis-associated bacteria); and/or the disease-associated bacterium is selected from the group consisting of *Atopobium* spp., and *Oribacterium* spp (caries-associated bacteria).

In embodiments of the composition for use of the invention, the composition is topically applied or ingested.

In embodiments of the composition for use of the invention, the composition is selected from the group consisting of toothpaste, mouthwash, oral gel, dental floss, tongue paste, food extract, chewing gum, chewing tablet, supplement powder, tablet, pill, capsule, pet food, livestock food, starch-containing product, sugar-containing product, food, sport drink, sport snack, gummy, and candy.

In embodiments of the composition for use of the invention, the composition comprises a further ingredient useful for the treatment and/or prevention of oral diseases and/or potential enhancers of the effects of glutamate, such as arginine, nitrate, ammonium, aspartate, and bicarbonate.

Nitrate is a prebiotic that prevent caries, periodontitis and halitosis. Nitrate is converted into nitrite, which can be further reduced into ammonium (which buffers acidic pH) or antimicrobial nitric oxide (which inhibits the growth of oral pathogens). During these reduction processes, lactic acid and a volatile compound associated with halitosis, hydrogen sulphide, are metabolized by nitrate-reducing bacteria, limiting halitosis. Additionally, health-associated bacteria increase in the presence of nitrate, while the levels of caries-, periodontitis- and halitosis-associated bacteria decrease. Arginine, ammonium, aspartate, glutamine, and bicarbonate are other ingredients useful for the treatment and/or prevention of oral diseases and/or potential enhancers of the effects of glutamate.

Therefore, in embodiments of the composition for use of the invention, the composition comprises a further ingredient selected from the group consisting of arginine, nitrate, ammonium, aspartate, glutamine, and bicarbonate.

The present invention also provides an *in vitro* method to determine the response of a mammal to the treatment with glutamate, or with an amine, solvate or hydrate thereof, the method comprising:
(a) determining at least one parameter selected from the group consisting of: amount of at least one health-associated bacterium in oral biofilms of the mammal, amount of at least one disease-associated bacterium in oral biofilms of the mammal, and dysbiosis index, in a saliva sample obtained from a mammal after said mammal have been administered with an oral composition comprising glutamate, or of the amine, solvate or hydrate thereof, and
(b) identifying the mammal as responsive to the treatment with the composition if any one of the following conditions is fulfilled:
   (i) the amount of at least one health-associated bacterium increases, with respect to the amount before treatment;
   (ii) the amount of at least one disease-associated bacterium decreases, with respect to the amount before treatment;
   (iii) the value of the dysbiosis index decreases, with respect to the dysbiosis index before treatment,
or identifying the mammal as non-responsive to the treatment with the composition if any one of the following conditions is fulfilled:
(i) the amount of at least one health-associated bacterium is equal to or decreases, with respect to the amount before treatment;
(ii) the amount of at least one disease-associated bacterium is equal to or decreases, with respect to the amount before treatment;
(iii) the value of the dysbiosis index is equal to or increases, with respect to the dysbiosis index before treatment.

In embodiments of the method of the invention, the mammal is a human.

In embodiments of the method of the invention, the dysbiosis index is the Subgingival Microbial Dysbiosis Index (SMDI).

In embodiments of the method of the invention, when the mammal is identified as responsive, the composition is useful for the treatment and/or prevention of an oral disease in the mammal, wherein the oral disease is selected from periodontal disease, halitosis, and oral disease caused by *Candida.*

In embodiments of the method of the invention, the health-associated bacterium is selected from the group consisting of *Haemophilus haemolyticus, Haemophilus pittmaniae, Haemophilus parainfluenzae, Haemophilus* spp., *Actinomyces* spp., *Veillonella rogosae, Veillonella dispar,* and *Veillonela* spp.; and/or the disease-associated bacterium is selected from the group consisting of *Porphyromonas endodontalis, Porphyromonas* spp., *Treponema* spp., *Catonella morbi, Catonella* spp., *Filifactor alocis, Filifactor* spp*., Prevotella intermedia, Prevotella* spp., *Dialister invisus, Dialister* spp., *Absconditabacteriales* (SR1) spp., *Alloprevotella tannerae, Alloprevotella* spp., *Megasphaera micronuciformis, Megasphaera* spp., *Atopobium parvulum, Atopobium* spp., *Oribacterium parvum,* and *Oribacterium spp.*

In embodiments of the method of the invention, the glutamate is selected from the group consisting of monosodium glutamate, potassium glutamate, calcium glutamate, magnesium glutamate, and ammonium glutamate.

In embodiments of the method of the invention, the amine is glutamine.

As used herein, the term "comprise", "comprising" and their variants includes, specifically, the term "consisting" or "consisting of".

As used herein, the singular forms "a", "an", and "the" include plural referents unless the content clearly dictates otherwise.

### BRIEF DESCRIPTION OF DRAWINGS

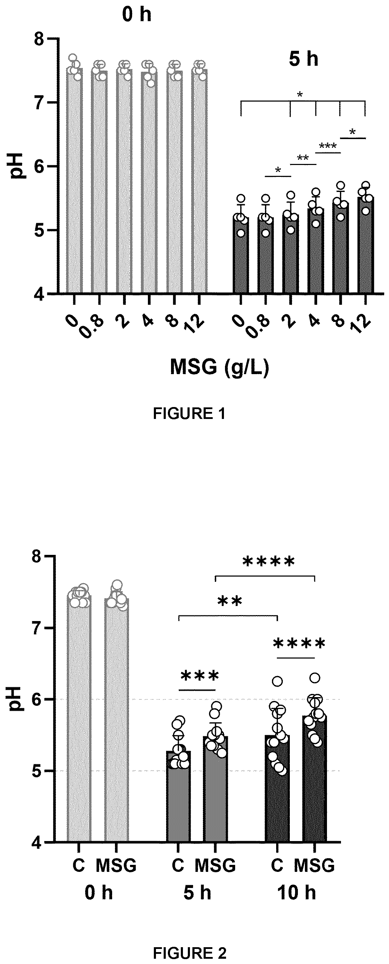

**Figure 1****.** Effect of monosodium glutamate supplementation on pH. Barplots show averages and standard deviation of measurements in supernatants from 5 donors at baseline (0 h, light grey bars) and after 5 h of incubation (dark grey bars) with 2 g/L sugar addition. In the x-axis, the concentration of monosodium glutamate is shown in g/L. Circles represent individual samples. *p < 0.05, **p < 0.01, ***p < 0.001, ****p < 0.0001 according to a paired t-test. MSG: monosodium glutamate.
**Figure 2****.** Effect of monosodium glutamate (MSG) supplementation on pH. Barplots show averages and standard deviation of measurements in supernatants from 13 donors under 10 g/L monosodium glutamate and control conditions at baseline (0 h, light grey bars) and after 5 h (grey bars) and 10 h (dark grey bars) of incubation. The 0 h bars represent the average pH of the medium mixed with saliva before incubation. Circles represent the different donors at 5 h and 10 h. **p < 0.01, ***p < 0.001, ****p < 0.0001 according to a paired t test or Wilcoxon test. C: control condition. MSG: Monosodium glutamate condition (10 g/L).

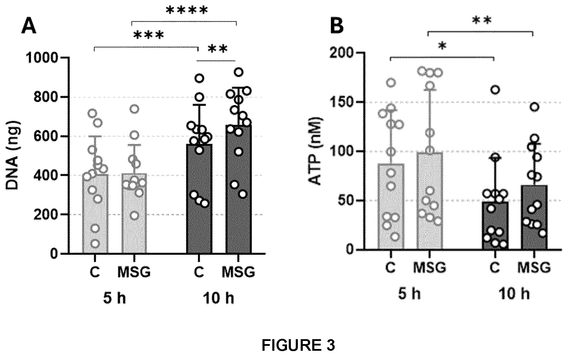

**Figure 3****.** Effect of 10 g/L monosodium glutamate (MSG) supplementation on biofilm. (A) DNA. (B) Absolute ATP (a proxy of cell viability). Barplots show averages and standard deviation of measurements in biofilms from 12 donors under 10 g/L monosodium glutamate and control conditions at different times of incubation (5 h and 10 h). Circles represent different donors. *p < 0.05, **p < 0.01, ***p < 0.001, ****p < 0.0001 according to a paired t-test or Wilcoxon test. C: control condition. MSG: Monosodium glutamate condition (10 g/L). DNA (ng): the amount of DNA per biofilm in nanograms. ATP: Adenosine triphosphate. nM: nanomolar.

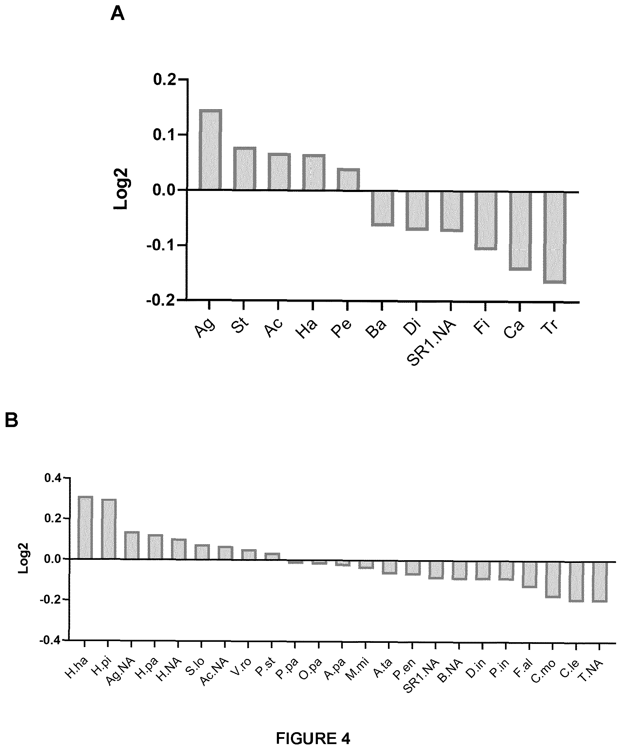

**Figure 4****.** Log2 fold-change of all significant genera (A) and species (B) (p-value < 0.05). Apart from significant changes in bacterial genera, clinically relevant *Dialisterwas* added (p < 0.07). Apart from significant changes in species, clinically relevant *Veillonella rogosae, Prevotella intermedia, Megasphaera micronuciformis, Oribacterium parvum* and *Prevotella pallens* were added (all p < 0.06). Bars values indicate organisms that increased or decreased significantly in the monosodium glutamate condition compared to the control condition. n = 13. The relative abundances transformed with ANCOM-BC were used. Log2: the log2 value of the transformed relative abundance of the monosodium glutamate condition divided by the transformed relative abundance of the control condition. The relative abundances before transformation can be found in **Table 1** and **Table 2.** The following abbreviations have been used in this Figure: Ag: *Aggregatibacter;* St: *Stomatobaculum;* Ac: *Actinomyces;* Ha: *Haemophilus;* Pe: *Peptostreptococcus;* Ba: *Bacteroides;* Di: *Dialister;* SR1.NA: *Absconditabacteriales* (SR1) NA; Fi: *Filifactor;* Ca: *Catonella;* Tr: *Treponema;* H.ha: *Haemophilus haemolyticus;* H.pi: *Haemophilus pittmaniae;* Ag.NA: *Aggregatibacter* NA; H.pa: *Haemophilus parainfluenzae;* H.NA: *Haemophilus* NA; S.lo: *Stomatobaculum longum;* Ac.NA: *Actinomyces* NA; V.ro: *Veillonella rogosae;* P.st: *Peptostreptococcus stomatis;* P.pa: *Prevotella pallens;* O.pa: *Oribacterium parvum;* A.pa: *Atopobium parvulum;* M.mi: *Megasphaera micronuciformis;* A.ta: *Alloprevotella tannerae;* P.en: *Porphyromonas endodontalis;* SR1.NA: Absconditabacteriales (SR1) NA; B.NA: *Butyrivibrio* NA; D.in: *Dialister invisus;* P.in: *Prevotella intermedia;* F.al: *Filifactor alocis;* C.mo: *Catonella morbi;* C.le: *Capnocytophaga leadbetteri;* T.NA: *Treponema* NA.

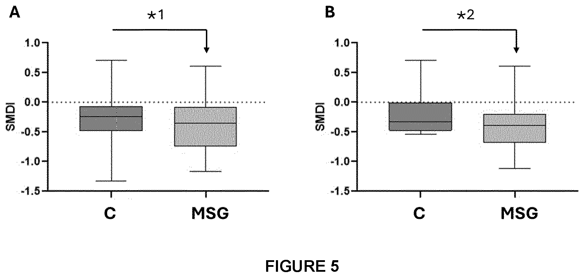

**Figure 5****.** Dysbiosis associated with periodontitis is decreased by glutamate. The Subgingival Microbial Dysbiosis Index (SMDI), which can also be used for saliva, was calculated using the online platform to determine the dysbiosis in our saliva-derived biofilms. *¹ Glutamate decreased the dysbiosis index from -0.27 to -0.36 on average in all 13 individuals. *² In 3 individuals, glutamate did not have this effect and after removal of these 3 'non-responders', the dysbiosis index significantly decreased from -0.19 to 0.40 (p < 0.05) in the remaining 10 individuals. SMDI: Subgingival Microbial Dysbiosis Index. MSG: monosodium glutamate. C: control.

### DESCRIPTION OF EMBODIMENTS

### Example 1. Materials and methods

### Unstimulated saliva sampling

Each of the healthy donors who have participated in the experiments were instructed not to eat breakfast or brush their teeth before saliva collection in the morning. Depending on the experiment, different amounts of unstimulated saliva (2-5 mL) were collected by drooling in a sterile tube. The fresh unstimulated saliva was directly used for *in vitro* growth. All participants signed an informed consent prior to sample donation.

### In vitro oral biofilm growth

100 µL of unstimulated saliva from each donor were grown in standard 96-well plate with 150 µL of culture medium. The culture media used was Brain Heart Infusion (BHI) oxoid medium (Thermo Fisher Scientific, Waltham, Massachusetts, USA), supplemented with 5 mg/L hemin and 1 mg/L menadione (all Sigma-Aldrich, St. Louis, Missouri, USA). In addition, depending on the experiment, the culture media were supplemented with different concentrations of sucrose (Condalab, Torrejón de Ardoz, Madrid, Spain), and L-Glutamic acid monosodium salt monohydrate 98+% (Thermo Fisher Scientific, Waltham, Massachusetts, USA). Plates were sealed with adhesive aluminium foil (VWR, Radnor, Pennsylvania, USA) to prevent oxygen diffusion and depending on the experiment the plates were incubated for 0, 5, 10 or 12 h at 37 °C. After the incubation period, the supernatant from each well was sampled and stored at - 20 °C until measurements. After removing supernatant, the biofilms were resuspended in 50 µL PBS for storage at - 80 °C to avoid degradation of the genetic material.

### pH measurements

All supernatants collected from the *in vitro* biofilms at different time points were used to determine the pH levels using the RQflex 10 Reflectoquant reflectometer (Merck Milipore, Burlington, Massachusetts, USA) and the pH test strips (reference no. 1169960001). For this, 15 µL of supernatant was pipetted on each of the two reactive patches of a pH strip.

### Bacterial DNA extraction

For DNA extraction, biofilm duplicates from the final experiment were combined to obtain 100 µL, of which 10 µL was stored at -80 °C for ATP quantification and 90 µL was used for DNA extraction. DNA was extracted from biofilms using the MasterPure Complete DNA and RNA purification Kit (Epicentre Biotechnology, Madison, Wisconsin, USA) following the manufacturer's instructions, with the addition of lysozyme. The isolated DNA/RNA was resuspended in 32 µL H₂0 miliQ and stored at -80°C in a volume of 12 µL for Illumina sequencing of the 16S rRNA gene (DNA) and 20 µL for future RNA analysis.

### ATP quantification

ATP was measured to quantify cell viability indirectly using the BacTiter-GloTM Microbial Cell Viability Assay (Promega, Madison, Wisconsin, USA), according to manufacturer's instructions.

### DNA quantification

DNA concentration was measured using the Qubit 1 × dsDNA HS Assay Kit and a Qubit 3 Fluorometer (both Thermo Fisher Scientific, Waltham, Massachusetts, USA), according to manufacturer's instructions.

### Biofilm composition determined by 16 rRNA sequencing

DNA from biofilms obtained during the final experiment, which were incubated for 10 h, was sequenced to determine the bacterial composition. For sequencing, an Illumina amplicon library was performed following the 16S rRNA gene Metagenomic Sequencing Library Preparation Illumina protocol (Part #15044223 Rev.A). The primer sequences used in this protocol target the 16S V3 and V4 region. Following amplification, DNA was sequenced with an Illumina MiSeq Sequencer according to manufacturer's instructions using the 2 × 300 base paired-ends protocol.

### Taxonomic classification

The DADA2 pipeline (v1.8) in R was used to process the paired-end FASTQ files. The forward and reverse reads were trimmed, removing the primer sequences and low-quality bases at the end of the reads trough end-trimming. Reads with any ambiguous N base or exceeding 5 expected errors were also discarded. The forward and reverse pairs were combined, with a minimum overlap of 12 bases and a maximum mismatch of 1 base in the overlapping region, to obtain the single denoised variants. After chimera removal, the final amplicon sequence variants (ASVs) were mapped onto the Homo sapiens genome (assembly GRCh38.p13), using Bowtie (v2.3.5.1), in order to remove artefactual reads from the host. The Silva database (v138) was set as a reference to assign taxonomy to each ASV. Genus classification was achieved using the DADA2 naive Bayesian classifier method. The ASVswith an assigned genus but without exact species, were aligned using the Blastn tool (v2.10.0+) against the Silva database with a minimum of 97% of identity. The relative abundance of bacterial genera and species was determined. Before statistical analyses, bacterial abundance was transformed using ANCOM-BC approach to account for the compositional nature of 16 S data. The transformed relative abundances were compared with a Wilcoxon test. The Subgingival Microbial Dysbiosis Index (SMDI), which can also be used for saliva, was calculated using the online platform (bioinformatics.forsyth.org/smdi, visited on 18th July 2024 using the standard settings) to determine the dysbiosis in our saliva-derived biofilms.

### Statistical analysis

Statistical analysis was performed using Student's t-test or a non-parametric Wilcoxon test, depending on whether the data followed a normal distribution or not, using GraphPad Prism (version 10.0.2) and were considered statistically significant at p-value < 0.05.

### Example 2. Assays with glutamate in saliva samples

### Effect of glutamate on oral pH

A first experiment was performed with saliva of donors (n=5) to which medium with 2 g/L extra sucrose was added and different monosodium glutamate concentrations (0.8, 2, 4, 8 and 12 g/L), as well as control without monosodium glutamate (**Figure 1**). At 5 h, significant differences were observed between the 0 g/L condition and all other conditions except the lowest monosodium glutamate condition (i.e., 0.8 g/L monosodium glutamate). In addition, there were also significant differences between the monosodium glutamate conditions (the more monosodium glutamate, the higher the pH, all p-value < 0.05).

A second experiment was performed with saliva of donors (n = 13) to which medium with 2 g/L extra sucrose was added and 2 conditions (control and 10 g/L monosodium glutamate) were compared at 3 time points (0 h, 5 h and 10 h) (**Figure 2**). A significant increase in pH (p < 0.001) was seen in the monosodium glutamate condition after 5 h and 10 h of incubation. At 5 h, the control condition had a mean pH value of 5.28, while the 10 g/L monosodium glutamate condition had a mean pH of 5.48 (p = 0.0002). Similarly, at 10 h the control condition had a mean pH value of 5.5, while the 10 g/L monosodium glutamate condition had a mean pH of 5.77 (p < 0.0001), keeping the pH above the critical level in which enamel is demineralized (i.e., around pH 5.5). Significant changes were also seen comparing the 5 h and 10 h timepoint of each condition. Specifically, the pH of both conditions is higher at 10 h than at 5 h (p < 0.01 for the control condition and p < 0.0001 for the monosodium glutamate condition).

### Effect of glutamate on biofilm growth

DNA concentration in the biofilms was used as a proxy of cell numbers. The addition of 10 g/L monosodium glutamate did not result in a significant difference in DNA at 5 h; however, at 10 h significantly more DNA was detected in the monosodium glutamate condition (p < 0.01) (**Figure 3A**). This indicates that glutamate stimulates the growth of certain bacteria. In addition, more DNA was detected in the control and monosodium glutamate conditions at 10 h compared to 5 h (both p < 0.001), showing that there was biofilm growth in this short incubation period.

### Effect of glutamate on cell viability

There was higher ATP concentration (an indication of cell viability) detected at 5 h than at 10 h (p < 0.05 for control condition and p < 0.01 for monosodium glutamate condition) (**Figure 3B**), indicating that as biofilms become more mature and thicker, less ATP is produced. The addition of 10 g/L monosodium glutamate did not affect the total amount of ATP at both timepoints (5 h and 10 h) (**Figure 3B**), suggesting that monosodium glutamate did not affect cell viability.

### Effect of glutamate on in vitro oral biofilm composition

The relative abundances of the bacterial composition of the 10 h *in vitro* biofilms were determined as average percentages to represent the bacterial composition in the absence or presence of monosodium glutamate on a genus-level (**Table 1**) or species-level (**Table 2**). **Table 1** shows dominant genera composition (genera with >1% relative abundance). **Table 2** shows dominant species composition (species with >1% relative abundance).

**Table 1**

| **Genus** | **10 g/L glutamate Relative abundance(%)** | **Control Relative abundance(%)** | **Ratio glutamate (%)/control (%)** |
|---|---|---|---|
| *Veillonella* | 31.4 | 30.2 | 1.04 |
| *Streptococcus* | 28.1 | 29.5 | 0.95 |
| *Prevotella* | 10.9 | 11.1 | 0.99 |
| *Neisseria* | 4.2 | 5.1 | 0.84 |
| *Haemophilus* | 5.6 | 4.3 | 1.29** |
| *Alloprevotella* | 3.8 | 4.0 | 0.96 |
| *Porphyromonas* | 1.7 | 1.9 | 0.87 |
| *Gemella* | 1.3 | 1.6 | 0.81 |
| *Megasphaera* | 1.0 | 1.2 | 0.85 |
| *Rothia* | 1.2 | 1.2 | 1.03 |
| *Fusobacterium* | 1.2 | 1.1 | 1.15 |
| *Peptostreptococcus* | 1.1 | 0.7 | 1.58* |
| *Actinomyces* | 1.2 | 0.7 | 1.77** |
| Other | 7.2 | 7.4 | |
| *p < 0.05 comparing the two conditions after relative abundance transformation with ANCOM-BC using a Wilcoxon test | | | |
| **p < 0.01 comparing the two conditions after relative abundance transformation with ANCOM-BC using a Wilcoxon test | | | |

**Table 2**

| **Species** | **10 g/L glutamate Relative abundance(%)** | **Control Relative abundance (%)** | **Ratio glutamate (%)/control (%)** |
|---|---|---|---|
| *Streptococcus* NA | 27.5 | 28.9 | 0.95 |
| *Veillonella atypica* | 8.9 | 9.6 | 0.93 |
| *Veillonella* NA | 7.0 | 8.4 | 0.83 |
| *Veillonella dispar* | 9.0 | 7.3 | 1.23 |
| *Prevotella melaninogenica* | 3.9 | 4.7 | 0.84 |
| *Neisseria* NA | 4.3 | 4.2 | 1.01 |
| *Veillonella tobetsuensis* | 3.9 | 3.2 | 1.24 |
| *Alloprevotella* NA | 2.8 | 2.9 | 0.97 |
| *Haemophilus* NA | 2.5 | 2.5 | 1.00 (1.10 after ANCOM-BC transformation)* |
| *Prevotella* NA | 3.3 | 2.3 | 1.43 |
| *Porphyromonas pasteri* | 1.4 | 1.6 | 0.90 |
| *Haemophilus sputorum* | 1.1 | 1.3 | 0.78 |
| *Prevotella salivae* | 1.2 | 1.3 | 0.88 |
| *Prevotella pallens* | 1.0 | 1.2 | 0.85^{#} |
| *Megasphaera micronuciformis* | 1.1 | 1.2 | 0.93^{#} |
| *Fusobacterium periodonticum* | 1.1 | 1.2 | 0.90 |
| *Gemella* NA | 1.7 | 1.1 | 1.48 |
| *Veillonella rogosae* | 1.1 | 0.7 | 1.58^{#} |
| Other | 17.3 | 16.4 | |
| ^{#}p = 0.05-0.06 comparing the two conditions after relative abundance transformation with ANCOM-BC using a Wilcoxon test | | | |
| *p < 0.05 comparing the two conditions after relative abundance transformation with ANCOM-BC using a Wilcoxon test | | | |

In the control condition, the five most abundant genera were *Veillonella, Streptococcus, Prevotella, Neisseria* and *Haemophilus* (**Table 1**).

In the 10 g/L monosodium glutamate condition, the five most abundant genera were the same as in the control, but *Haemophilus* was more abundant than *Neisseria.* The order in this condition was: *Veillonella, Streptococcus, Prevotella, Haemophilus* and *Neisseria* (**Table 1**).

In the control condition, the five most abundant species were: *Streptococcus* sp., *Veillonella atypica, Veillonella* NA (NA being sequences that could not be assigned on a species-level, i.e., unclassified *Veillonella* species), *Veillonella dispar* and *Prevotella melaninogenica* (**Table 2**). In the monosodium glutamate condition, these were *Streptococcus* NA, *Veillonella dispar, Veillonella atypica, Veillonella* NA and *Neisseria* NA.

The monosodium glutamate condition was compared with the control condition and 10 and 18 significant changes (p < 0.05) were observed on a genus- and species-level, respectively, in the 10 h *in vitro* biofilms. Of the 10 genera, 5 increased (*Aggregatibacter, Stomatobaculum, Actinomyces, Haemophilus* and *Peptostreptococcus*) and 5 decreased (*Bacteroides, Absconditabacteriales* (SR1) NA, *Filifactor, Catonella* and *Treponema*) in abundance (**Figure 4A**). Additionally, periodontitis-associated *Dialister* decreased (p < 0.07). At the species level, 8 species increased and 10 decreased in abundance resulting from monosodium glutamate supplementation (**Figure 4B**). The species that increased were *Haemophilus haemolyticus, Haemophilus pittmaniae, Aggregatibacter* NA (this was not the *periopathogenic Aggregatibacter actinomycetemcomitans,* which was detected seperately), *Haemophilus parainfluenzae, Haemophilus* NA, *Stomatobaculum longum, Actinomyces* NA, *Peptostreptococcus stomatis.* The decreasing species included *Treponema* NA, *Capnocytophaga leadbetteri, Catonella morbi, Filifactor alocis, Dialister invisus, Butyrivibrio* NA, *Absconditabacteriales* (SR1) NA, *Porphyromonas endodontalis, Alloprevotella tannerae, Atopobium parvulum.*

Additionally, *Veillonella rogosae* increased, while *Prevotella pallens, Oribacterium parvum, Megasphaera micronuciformis* and *Prevotella intermedia* decreased (all p < 0.06).

The Subgingival Microbial Dysbiosis Index (SMDI), which can also be applied to saliva samples, was calculated. This index represents the degree of dysbiosis from a periodontitis point of view. Glutamate decreased the dysbiosis index from -0.27 to -0.36 on average in all 13 individuals (**Figure 5A**). In 3 individuals, glutamate did not have this effect and after removal of these 3 "non-responders", the dysbiosis index significantly decreased from -0.19 to 0.40 (p < 0.05) in the remaining 10 individuals (**Figure 5B**). This indicates that glutamate may be more effective in some individuals than others. SMDI: Subgingival Microbial Dysbiosis Index. MSG: monosodium glutamate. C: control.

## Claims

1. An oral composition comprising glutamate, or an amine, solvate or hydrate thereof, for use in the treatment and/or prevention of caries in a mammal, wherein the composition is orally administered to the mammal and increases the pH of the saliva of the mouth of the mammal, thereby, reducing the risk of caries development in the mammal.

2. The composition for use according to claim 1, wherein the glutamate is selected from the group consisting of monosodium glutamate, potassium glutamate, calcium glutamate, magnesium glutamate, and ammonium glutamate.

3. The composition for use according to claim 1, wherein the amine is glutamine.

4. The composition for use according to any one of claims 1 to 3, wherein the concentration of glutamate, or of the amine, solvate or hydrate thereof, in the composition, is from 10 to 210 mmol/kg.

5. The composition for use according to any one of claims 1 to 4, wherein the concentration of glutamate, or of the amine, solvate or hydrate thereof, in the composition, is from 10 to 70 mmol/kg.

6. The composition for use according to any one of claims 1 to 5, wherein the composition increases the pH by consuming protons in decarboxylation reaction of glutamate to produce γ-aminobutyric acid (GABA) catalysed by the enzyme glutamate decarboxylase.

7. The composition for use according to any one of claims 1 to 6, wherein the composition further increases the amount of at least one health-associated bacterium in oral biofilms of the mammal and/or decreases the amount of at least one disease-associated bacterium in oral biofilms of the mammal, wherein the composition is useful for the treatment and/or prevention of an oral disease in the mammal, wherein the oral disease is selected from periodontal disease, halitosis, and oral disease caused by *Candida.*

8. The composition for use according to claim 7, wherein the health-associated bacterium is selected from the group consisting of *Haemophilus haemolyticus, Haemophilus pittmaniae, Haemophilus parainfluenzae, Haemophilus* spp., *Actinomyces* spp., *Veillonella rogosae, Veillonella dispar,* and *Veillonela* spp.; and/or the disease-associated bacterium is selected from the group consisting of *Porphyromonas endodontalis, Porphyromonas* spp., *Treponema* spp., *Catonella morbi, Catonella* spp., *Filifactor alocis, Filifactor* spp., *Prevotella intermedia, Prevotella* spp., *Dialister invisus, Dialisterspp., Absconditabacteriales* (SR1) spp., *Alloprevotella tannerae, Alloprevotella* spp., *Megasphaera micronuciformis, Megasphaera* spp., *Atopobium parvulum, Atopobium* spp., *Oribacterium parvum,* and *Oribacterium spp.*

9. The composition for use according to any one of claims 1 to 8, wherein the composition is topically applied or ingested.

10. The composition for use according to any one of claims 1 to 9, wherein the composition is selected from the group consisting of toothpaste, mouthwash, oral gel, dental floss, tongue paste, food extract, chewing gum, chewing tablet, supplement powder, tablet, pill, capsule, pet food, livestock food, starch-containing product, sugar-containing product, food, sport drink, sport snack, gummy and candy.

11. The composition for use according to any one of claims 1 to 10, wherein the composition comprises a further ingredient selected from the group consisting of arginine, nitrate, ammonium, aspartate, and bicarbonate.

12. An *in vitro* method to determine the response of a mammal to the treatment with glutamate, or with an amine, solvate or hydrate thereof, the method comprising:
(a) determining at least one parameter selected from the group consisting of: amount of at least one health-associated bacterium in oral biofilms of the mammal, amount of at least one disease-associated bacterium in oral biofilms of the mammal, and dysbiosis index, in a saliva sample obtained from a mammal after said mammal have been administered with an oral composition comprising glutamate, or with an amine, solvate or hydrate thereof, and
(b) identifying the mammal as responsive to the treatment with the composition if any one of the following conditions is fulfilled:
(i) the amount of at least one health-associated bacterium increases, with respect to the amount before treatment;
(ii) the amount of at least one disease-associated bacterium decreases, with respect to the amount before treatment;
(iii) the value of the dysbiosis index decreases, with respect to the dysbiosis index before treatment,
or identifying the mammal as non-responsive to the treatment with the composition if any one of the following conditions is fulfilled:
(i) the amount of at least one health-associated bacterium is equal to or decreases, with respect to the amount before treatment;
(ii) the amount of at least one disease-associated bacterium is equal to or decreases, with respect to the amount before treatment;
(iii) the value of the dysbiosis index is equal to or increases, with respect to the dysbiosis index before treatment.

13. The method according to claim 12, wherein the dysbiosis index is the Subgingival Microbial Dysbiosis Index (SMDI).

14. The method according to claim 12 or 13, wherein when the mammal is identified as responsive, the composition is useful for the treatment and/or prevention of an oral disease in the mammal, wherein the oral disease is selected from periodontal disease, halitosis, and oral disease caused by *Candida.*

15. The method according to any one of claims 12 to 14, wherein the health-associated bacterium is selected from the group consisting of *Haemophilus haemolyticus, Haemophilus pittmaniae, Haemophilus parainfluenzae, Haemophilus* spp., *Actinomyces* spp., *Veillonella rogosae, Veillonella dispar,* and *Veillonela* spp.; and/or the disease-associated bacterium is selected from the group consisting of *Porphyromonas endodontalis, Porphyromonas* spp., *Treponema* spp., *Catonella morbi, Catonella* spp., *Filifactor alocis, Filifactor* spp., *Prevotella intermedia, Prevotella* spp., *Dialister invisus, Dialister* spp., *Absconditabacteriales* (SR1) spp., *Alloprevotella tannerae, Alloprevotella* spp., *Megasphaera micronuciformis, Megasphaera* spp., *Atopobium parvulum, Atopobium* spp., *Oribacterium parvum,* and *Oribacterium spp.*
